**Europäisches Patentamt**

**European Patent Office**

**Office européen des brevets**

(11) Numéro de publication: **0 100 730**
**B1**

(12) **FASCICULE DE BREVET EUROPEEN**

(45) Date de publication du fascicule du brevet:
**17.09.86**

(21) Numéro de dépôt: **83401568.7**

(22) Date de dépôt: **28.07.83**

(51) Int. Cl.⁴: **A 01 M 1/20,** A 01 M 13/00,
A 61 L 9/12

(54) Dispositif diffuseur de substances volatiles, notamment d'insecticide.

(30) Priorité: **03.08.82 FR 8213575**

(43) Date de publication de la demande:
**15.02.84 Bulletin 84/7**

(45) Mention de la délivrance du brevet:
**17.09.86 Bulletin 86/38**

(84) Etats contractants désignés:
**AT BE CH DE GB IT LI NL SE**

(56) Documents cités:
**CH - A - 402 503**
**FR - A - 2 031 730**
**FR - A - 2 060 219**
**FR - A - 2 063 960**
**GB - A - 2 078 112**
**US - A - 1 915 583**
**US - A - 2 616 759**
**US - A - 4 310 985**

(73) Titulaire: **L'OREAL, 14, Rue Royale, F-75008 Paris (FR)**

(72) Inventeur: **Goncalves, Antonin, 41, rue du Lac Marchais, F-95410 Groslay (FR)**

(74) Mandataire: **Peuscet, Jacques, Cabinet Peuscet 68, rue d'Hauteville, F-75010 Paris (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne un dispositif diffuseur de substances volatiles notamment d'insecticide, consistant en un réceptacle à l'intérieur duquel est disposée une plaquette constituée par un support absorbant imprégné d'un produit actif à évaporation lente, qui, dans le cas des diffuseurs d'insecticide, peut être un mélange d'un composé actif tel que le diméthyl-dichlorovinylphosphate, et d'un liquide à viscosité élevé tel qu'un terpène chloré, le produit actif s'évaporant dans l'atmosphère, à travers des évidements pratiqués dans les parois du réceptacle.

Les réceptacles pour plaquettes insecticides connus sont généralement des réceptacles ayant la forme de parallélépipèdes plats qui sont réalisés en deux parties identiques, s'assemblant l'une sur l'autre dans le plan longitudinal médian du réceptacle, par exemple comme cela est décrit dans le brevet français n° 2 031 730. Conformément à ce brevet, les deux parties comportent, en combinaison, d'une part, des organes mâles et femelles présents sur chacune des deux parties de manière à les positionner relativement l'une et l'autre par emboîtement réciproque, et, d'autre part, des organes en forme de crochets qui permettent l'encliquetage des deux parties l'une sur l'autre.

Le conditionnement de ces dispositifs diffuseurs est, à l'échelle industrielle, effectué en continu, les plaquettes absorbantes déshydratées étant imprégnées du mélange insecticide et mise en place dans les réceptacles de façon automatisée. On a proposé, pour ce conditionnement, une machine automatique assez compliquée et relativement coûteuse, qui a été décrite dans le brevet français 2 060 219. Il est apparu cependant que, même avec cette machine automatique, la lenteur de l'imprégnation des plaquettes par le mélange insecticide limitait considérablement les cadences de fabrication de ces dispositifs diffuseurs d'insecticide, alors que ces cadences devraient être rapides en raison de la large diffusion commerciale des produits concernés et du faible coût de conditionnement tolérable pour l'ensemble formé par le réceptacle et sa plaquette insecticide.

La présente invention a pour but de proposer un dispositif diffuseur d'insecticide permettant une imprégnation rapide de la plaquette insecticide qu'il contient, ce qui conduit à un gain de temps appréciable en fabrication.

Selon l'invention, on réalise l'imprégnation de la plaquette absorbante, alors que celle-ci est en place à l'intérieur du réceptacle, contrairement à la technique antérieure suivant laquelle l'imprégnation est effectuée avant le conditionnement de la plaquette dans le réceptacle. De plus, conformément à la présente invention, l'imprégnation de la plaquette est effectuée par capillarité, depuis la bordure supérieure de la plaquette, ce qui permet de supprimer tout risque d'égouttage. Pour assurer de cette manière l'imprégnation de la plaquette, le réceptacle est de conception originale. En effet, selon l'invention, on forme dans la partie supérieure du réceptacle disposé dans le sens vertical, un réservoir dont les parois internes sont constituées par des demi-cloisons formées chacune sur un élément de réceptacle, ces demi-cloisons maintenant ladite plaquette absorbante par pincement le long de leurs bordures libres.

L'étanchéité du réservoir est assurée par la plaquette absorbante ainsi pincée entre les demi-cloisons et la diffusion du mélange actif, que l'on verse dans le réservoir par un orifice prévu à cet effet dans la paroi supérieure du réceptacle, s'effectue par capillarité sans être bloquée par le pincement de la plaquette entre les demi-cloisons.

Cette double fonction assurée par le bord de la plaquette absorbante, dans la région de son échancrure, n'était pas évidente. On connaît, en effet, par le brevet anglais n° 2 078 112, un dispositif de vaporisation, notamment pour insecticides, constitué, d'une part, par un boîtier doté de perforations, supportant un buvard qui s'imprègne de substance active, laquelle s'évapore ensuite dans l'atmosphère, et, d'autre part, par une capsule remplie d'une matière absorbante contenant au départ les ingrédients actifs. Lorsque le dispositif est en service, la surface de la matière absorbante vient, en principe, en contact avec une région du buvard, les ingrédients actifs de la capsule passant graduellement dans le buvard. Une telle disposition n'est pas satisfaisante car, pour que l'imprégnation du buvard se fasse correctement, il faut que la capsule soit positonnée de façon très précise. Un tel problème ne se produit pas avec le dispositif diffuseur selon la présente invention.

La présente invention a donc pour objet le produit industriel nouveau que constitue un dispositif diffuseur comportant, à l'intérieur d'un réceptacle, au moins une plaquette de matériau absorbant susceptible d'être imprégnée par un produit actif liquide à évaporation lente, ledit réceptacle étant réalisé en deux éléments, qui s'assemblent l'un sur l'autre, et présentant des évidements destinés à assurer la circulation de l'air au contact de la plaquette disposée à l'intérieur du réceptacle, caractérisé par le fait qu'à l'intérieur du réceptacle est formé un réservoir situé à la partie supérieure du réceptacle en position de remplissage dudit réservoir et délimité, en premier lieu, par deux parois longitudinales en regard appartenant chacune à un des deux éléments assemblés pour former le réceptacle, en second lieu, par une paroi transversale du réceptacle dans laquelle est ménagé un orifice, et, en troisième lieu, par deux demi-cloisons appartenant chacune à un des deux éléments assemblés pour former le réceptacle, l'étanchéité dudit réservoir étant réalisée par la plaquette, qui, en position d'assemblage des deux éléments du réceptacle, est serrée entre les deux demi-cloisons, le produit actif liquide étant introduit par l'orifice dans le réservoir et diffusant de haut en bas dans le reste de la plaquette par capillarité.

La plaquette présente, de préférence, une échancrure sur son bord, qui se trouve adjacent à la paroi du récptacle comportant l'orifice par lequel on introduit le produit actif liquide.

Conformément à un mode de réalisation préféré du dispositif diffuseur selon l'invention, la paroi transversale du réceptacle, où est pratiqué l'orifice par lequel on introduit le produit actif liquide, est constituée de deux demi-parois transversales appartenant chacune à un des deux éléments dont l'assemblage forme le réceptacle; les deux parois longitudinales, qui délimitent le réservoir appartiennent chacune à un des deux éléments dont l'assemblage forme le réceptacle; l'assemblage des deux éléments du réceptacle s'effectue sensiblement dans le plan longitudinal moyen dudit réceptacle.

Conformément à un mode particulier de réalisation du dispositif diffuseur selon l'invention le réservoir ménagé à l'intérieur du réceptacle a une forme parallélépipédique, chaque demi-cloison étant constituée, en premier lieu, par une demi-bande parallèle à la demi-paroi transversale du réceptacle où est pratiqué l'orifice de remplissage du réservoir, cette demi-bande étant située à une certaine distance de cette demi-paroi transversale du réceptacle et, en second lieu, par deux demi-bandes perpendiculaires à la précédente, rejoignant la demi-paroi transversale du réceptacle.

Par ailleurs, on peut prévoir une échancrure dans chaque demi-paroi transversale du réceptacle destinée à constituer la paroi transversale portant ledit orifice, les deux échancrures reconstituant, lors de l'assemblage des deux éléments du réceptacle, l'orifice de remplissage.

Les deux parois longitudinales du réceptacle peuvent présenter des évidements de circulaiton d'air répartis sur toute leur surface, excepté dans la zone où se trouve le réservoir.

Les deux éléments du réceptacle s'assemblent l'un sur l'autre notamment par encliquetage, les organes d'encliquetage étant constitués par des crochets coopérant entre eux lorsque les demi-parois transversales des deux éléments du réceptacle viennent au contact l'une de l'autre.

A titre d'exemple, pour réaliser l'encliquetage, les bordures des demi-parois transversales du réceptacle, sauf celles des demi-parois comportant l'orifice de remplissage du réservoir, sont constituées sous la forme d'une pluralité de crochets tournés alternativement vers l'extérieur et vers l'intérieur du réceptable.

On peut également prévoir que les deux éléments du réceptacle sont réunis, le long des bordures de deux demi-parois transversales par une charnière-film venue de moulage, les bordures des autres demi-parois, qui sont destinées à coopérer entre elles, comportant des organes d'encliquetage. Comme produit actif, on peut utiliser notamment des produits insecticides, bactéricides, désodorisants et/ou parfumants.

Pour mieux faire comprendre l'objet de l'invention, on va en décrire ci-après, à titre d'exemple purement illustratif et non limitatif, un mode de réalisation représenté sur le dessin annexé.

Sur ce dessin:

– la figure 1 est une vue en perspective éclatée d'un dispositif diffuseur d'insecticide selon l'invention;

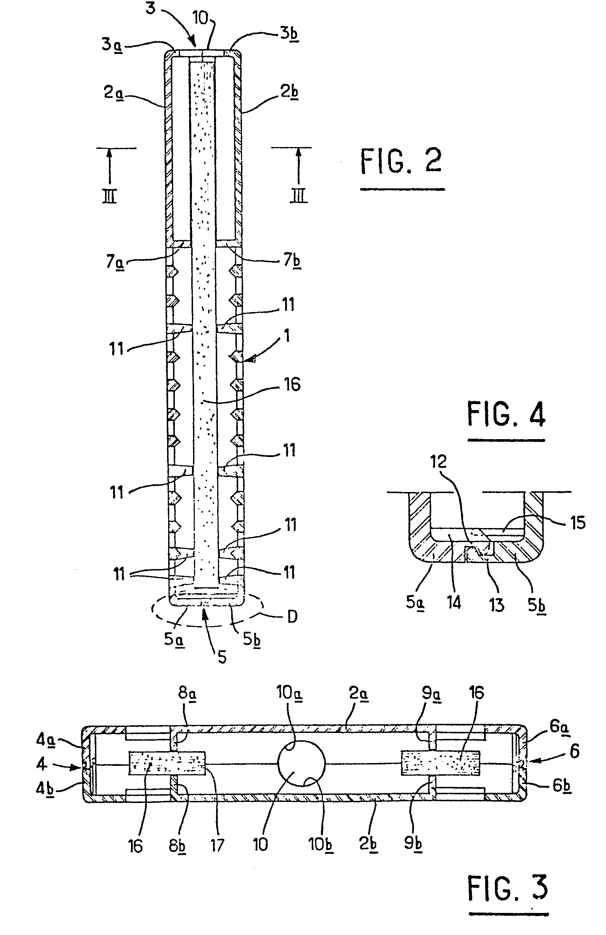

– la figure 2 est une vue en coupe verticale transversale du dispositif diffuseur après assemblage;

– la figure 3 est une vue en coupe selon III–III de la figure 2;

– la figure 4 est une vue agrandie de détail D de la figure 2, montrant les organes d'encliquetage des deux éléments formant chacun un demi-réceptacle.

Le dispositif diffuseur représenté sur le dessin comporte un réceptacle 1 qui, en position assemblée, a sensiblement la forme d'un parallélépipède plat, dont les deux parois longitudinales opposées portent le chiffre de référence 2a, 2b, alors que les parois transversales formant la périphérie du réceptacle et reliant entre elles les deux parois longitudinales ont été désignées par 3, 4, 5 et 6.

Le réceptacle 1 est divisé en deux éléments ou demi-réceptacles identiques 1a et 1b assemblés suivant un plan médian parallèle aux deux parois 2a, 2b. Les parois 3, 4, 5 et 6 du boîtier 1 sont constituées par le rapprochement des demi-parois correspondantes, à savoir respectivement: 3a, 3b; 4a, 4b; 5a, 5b et 6a, 6b.

Sur la face interne de chacune des parois 2a, 2b est ménagée, au voisinage de l'une des demi-parois transversales du réceptacle 1, en l'occurrence la demi-paroi respectivement 3a, 3b, une demi-cloison définie en premier lieu, par une demi-bande 7a, 7b parallèle à la demi-paroi transversale 3a, 3b et située à une certaine distance de cette demi-paroi et, en second lieu, par deux demi-bandes 8a, 9a; 8b, 9b, perpendiculaires à la précédente, rejoignant la demi-paroi transversale 3a, 3b du réceptacle 1.

En outre, chaque demi-paroi transversale 3a, 3b du réceptacle 1 présente, le long de sa bordure libre, une échancrure en demi-cercle 10a, 10b, les deux échancrures reconstituant, lors de l'assemblage des deux éléments 1a, 1b, du réceptacle 1, l'orifice 10 de remplissage du réservoir délimité par les deux parois longitudinales 2a, 2b, par la paroi transversale 3 et par les demi-cloisons 7a–8a–9a; 7b–8b–9b définies ci-dessus.

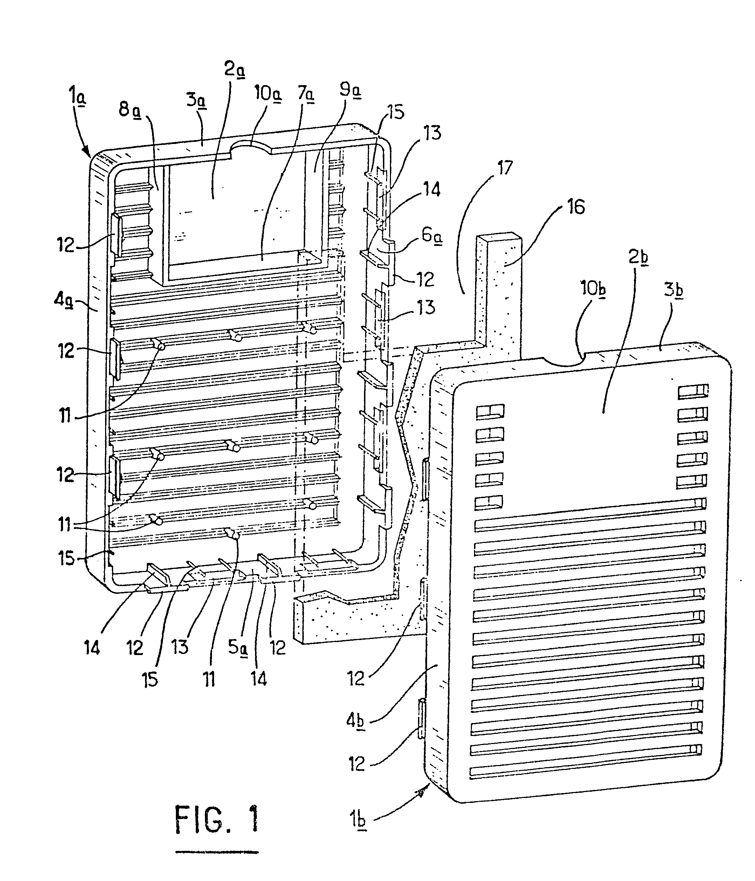

Les parois longitudinales 2a, 2b du réceptacle 1 présentent des évidements de circulation d'air répartis sur toute leur surface, excepté dans la zone où se trouve le réservoir, ces évidements étant constitués, comme on peut le voir sur la figure 1, par des lamelles formant jalousies, disposées obliquement par rapport au plan moyen desdites parois longitudinales 2a, 2b. En outre, les parois longitudinales 2a, 2b, comportent sur leur face interne, sauf dans la zone constituant le réservoir, des butées 11 dont le rôle est décrit plus loin.

Les deux éléments 1a, 1b, s'assemblent le long des bordures des demi-parois transversales 4a, 4b; 5a, 5b; 6a, 6b. A cet effet, on prévoit que ces bordures sont constitués sous la forme de crochets 12, 13 tournés alternativement respectivement vers l'extérieur et vers l'intérieur du réceptacle 1. Des nervures de rigidification 14 sont prévues sur les faces internes des demi-parois 4a, 4b; 5a, 5b; 6a, 6b, perpendiculairement aux crochets 12; par ailleurs, des petites tiges ou picots 15 parallèles aux demi-parois transversales précitées sont fixées sur les faces internes des parois 2a, 2b du réceptacle 1, à raison de deux tiges 15 devant chaque crochet 13. Ces tiges 15 aident au positionnement correct des éléments 1a, 1b lors de leur assemblage.

Le réceptacle 1 est destiné à recevoir une plaquette absorbante 16 qui peut être constituée par un feutre, une matière plastique frittée, un produit réalisé à partir de coton et d'amiante, et les produits analogues.

La plaquette absorbante 16, qui vient se placer dans le réceptacle 1 parallèlement aux parois 2a et 2b, a des dimensions telles qu'elle occupe sensiblement toute la surface intérieure du réceptacle 1. La plaquette 16 a donc une forme rectangulaire et présente avantageusement le long de son bord destiné à venir se placer en regard de la face 3 du réceptacle 1, une échancrure en U 17, entièrement logée à l'intérieur du réservoir que l'on a défini précédemment.

L'imprégnation de la plaquette insecticide 16 à l'aide d'un produit insecticide à évaporation lente s'effectue, selon l'invention, après son installation à l'intérieur du réceptacle 1. On dispose donc la plaquette 16, à l'état déshydraté, avec son échancrure 17 convenablement orientée, sur la paroi interne 2a ou 2b d'un élément 1a ou 1b et on vient fixer le second élément du réceptacle 1 sur le premier par encliquetage.

Dans le réceptacle assemblé, la plaquette absorbante 16 est maintenue par pincement entre les cloisons 7a–8a–9a, 7b–8b–9b, et sur le reste de sa surface, elle vient prendre appui sur les butées 11, dont la hauteur est déterminée en fonction de l'épaisseur de la plaquette absorbante 16.

Le réceptacle 1 étant alors placé sur sa paroi 5 opposée à la paroi 3 qui comporte l'orifice 10, le réservoir interne peut aisément être rempli avec le mélange d'imprégnation, le pincement de la plaquette absorbante 16 d'une part assurant l'étanchéité du réservoir et d'autre part, ne gênant pas la diffusion, par capillarité, dans la plaquette absorbante, du produit insecticide versé dans l'orifice 10.

A titre d'exemple de produits insecticides, on citera les dialkyl-phophates de vinyle, comme le diméthyldichlorophosphate de vinyle, leurs produits d'addition halogénés et des dialkyl-phosphorothioates de vinyle. Ces produits actifs sont avantageusement mélangés avec un produit à forte viscosité tel qu'un terpène chloré, ou une huile de ricin, l'évaporation du composé actif étant alors ralentie par la présence du liquide à forte viscosité.

A l'échelle de la fabrication industrielle, le processus d'imprégnation et de conditionnement est très facile, puisque l'on remplit les réservoirs formés à l'intérieur des réceptacles 1 de la même façon et à la même cadence que des flacons, le diamètre et le nombre de becs verseurs définissant une cadence de remplissage pouvant facilement être élevée. La répartition du produit dans la totalité du réceptacle se faisant par capillarité, il n'y a pas de risque d'égouttage. L'absorption du produit par une plaquette insecticide nécessite un temps approximatif de 5 minutes. Il suffit donc de laisser les réceptacles dans leur position verticale de remplissage pendant ce temps d'absorption relativement court, la mise sous sachet s'effectuant ensuite, lorsque le réservoir est tout à fait vide.

A la place des produits insecticides ci-dessus décrits, on peut utiliser:

– des produits désodorisants ou absorbeurs d'odeur tels que le fumarate d'hexyle ou le crotonate de géranyle dans un support à forte viscosité, tel que des glycols et leurs dérivés comme les éthers et les esters de glycol, des huiles minérales, comme la vaseline, des huiles végétales, comme l'huile de ricin, des esters d'acides gras, comme le myristate d'isopropyle, des nonyl-phénols oxyéthylènes (2 à 10 moles d'oxyde d'éthylène) et des phtalates de dioctyl.

– des produits parfumants, tels que: des parfums de pin, de pomme, de jasmin, de rose, de citron et de lavande dans un support à forte viscosité analogue à ceux désignés ci-dessus pour les produits désodorisants ou absorbeurs d'odeur.

## Revendications

1. Dispositif diffuseur comportant, à l'intérieur d'un réceptacle (1), au moins une plaquette (16) de matériau absorbant susceptible d'être imprégnée par un produit actif liquide à évaporation lente, ledit réceptacle (1) étant réalisé en deux éléments (1a, 1b) qui s'assemblent l'un sur l'autre et présentent des évidements destinés à assurer la circulation de l'air au contact de la plaquette (16) disposée à l'intérieur du réceptacle (1), caractérisé par le fait qu'à l'intérieur du réceptacle (1), est formé un réservoir situé à la partie supérieure du réceptacle (1) en position de remplissage dudit réservoir, et délimité, en premier lieu, par deux parois longitudinales en regard (2a, 2b) appartenant chacune à un des deux éléments (1a, 1b), assemblés pour former le réceptacle (1), en second lieu, par une paroi transversale (3) du réceptacle (1) dans laquelle est ménagé un orifice (10), et, en troisième lieu, par deux demi-cloisons (7a–8a–9a; 7b–8b–9b) appartenant chacune à un des deux éléments (1a, 1b) assemblés pour former le réceptacle (1), l'étanchéité dudit réservoir étant réalisée par la plaquette (16), qui, en position d'assemblage des deux éléments du réceptacle (1), est serrée entre les deux demi-cloisons (7a–8a–9a; 7b–8b–9b), le produit actif liquide étant introduit par l'orifice (10) dans le réservoir et diffusant de haut en bas dans le reste de la plaquette (16) par capillarité.

2. Dispositif diffuseur selon la revendication 1, caractérisé par le fait que la plaquette (16) présente une échancrure (17) sur son bord, qui se trouve adjacent à la paroi (3) du réceptacle comportant l'orifice (10).

3. Dispositif diffuseur selon l'une des revendications 1 et 2, caractérisé par le fait que l'assemblage des deux éléments du réceptacle (1) s'effectue sensiblement dans le plan longitudinal moyen dudit réceptacle (1).

4. Dispositif diffuseur selon l'une des revendications 1 à 3, caractérisé par le fait que la paroi transversale (3) du réceptacle, où est pratiqué l'orifice (10), est constituée de deux demi-parois transversales (3a, 3b) appartenant chacune à un des deux éléments (1a, 1b) dont l'assemblage forme le réceptacle (1).

5. Dispositif diffuseur selon la revendication 4, caractérisé par le fait que le réservoir ménagé à l'intérieur du réceptacle (1) a une forme parallélépipédique, chaque demi-cloison étant constituée, en premier lieu, par une demi-bande (7a ou 7b) parallèle à la demi-paroi transversale (3a ou 3b) du réceptacle (1) où est pratiqué l'orifice (10) et située à une certaine distance de cette demi-paroi transversale et, en second lieu, par deux demi-bandes (8a, 9a; 8b, 9b) perpendiculaires à la précédente, rejoignant la demi-paroi transversale (3a, 3b), du réceptacle (1).

6. Dispositif diffuseur selon la revendication 4, caractérisé par le fait que, dans chaque demi-paroi transversale (3a, 3b) du réceptacle (1) déstinée à constituer la paroi transversale (3) portant l'orifice (10) est prévue une échancrure (10a, 10b), les deux échancrures reconstituant, lors de l'assemblage des deux éléments du réceptacle (1), l'orifice (10) de remplissage.

7. Dispositif diffuseur selon l'une des revendications 1 à 6, caractérisé par le fait que les deux parois longitudinales (2a, 2b) du réceptacle (1) présentent des évidements de circulation d'air répartis sur toute leur surface, excepté dans la zone où se trouve le réservoir.

8. Dispositif diffuseur selon la revendication 7, caractérisé par le fait que les évidements des parois longitudinales (2a, 2b) sont constituées par des lamelles formant jalousies, disposées obliquement par rapport au plan moyen desdites parois longitudinales.

9. Dispositif diffuseur selon l'une des revendications 1 à 8, caractérisé par le fait que les parois longitudinales (2a, 2b) du réceptacle (1) comportent sur leur face interne, sauf dans la zone constituant le réservoir, des butées (11) sur lesquelles vient prendre appui la plaquette (16) lorsque le réceptacle (1) est assemblé.

10. Dispositif diffuseur selon l'une des revendications 1 à 9, caractérisé par le fait que les deux éléments (1a, 1b) du réceptacle (1) s'assemblent l'un sur l'autre par encliquetage, les organes d'encliquetage étant constitués par des crochets (12, 13) coopérant entre eux lorsque les demi-parois transversales des deux éléments (1a, 1b) du réceptacle (1) viennent au contact l'une de l'autre.

11. Dispositif diffuseur selon la revendication 10, caractérisé par le fait que les bordures des demi-parois transversales (4a, 4b; 5a, 5b; 6a, 6b) du réceptacle (1) sont constituées sous la forme d'une pluralité de crochets (12, 13) tournés alternativement vers l'extérieur et vers l'intérieur du réceptacle.

12. Dispositif diffuseur selon l'une des revendications 1 à 11, caractérisé par le fait que les deux éléments (1a, 1b) du réceptacle (1) sont réunis, le long des bordures de deux demi-parois transversales, par une charnière-film venue de moulage, les bordures des autres demi-parois, qui sont destinées à coopérer entre elles, comportant des organes d'encliquetage.

13. Dispositif diffuseur selon l'une des revendications 1 à 12, caractérisé par le fait que le produit actif liquide est un mélange à propriétés insecticides.

14. Dispositif diffuseur selon l'une des revendications 1 à 12, caractérisé par le fait que le produit actif est un mélange absorbeur d'odeurs à propriété désodorisantes ou parfumantes.

**Patentansprüche**

1. Verdampfungsvorrichtung mit mindestens einer Platte (16) aus absorbierendem Material im Inneren eines Behälters, wobei das Material mit einem langsam verdampfenden, aktiven flüssigen Produkt imprägniert werden kann und wobei der Behälter (1) aus zwei Elementen (1a, 1b) besteht, die aufeinander angeordnet sind und Ausnehmungen aufweisen zur Sicherstellung der Luftzirkulation in Kontakt mit der im Inneren des Behälters (1) angeordneten Platte (16), dadurch gekennzeichnet, dass im Inneren des Behälters (1) ein Reservoir ausgebildet ist, das sich beim Füllen im oberen Teil des Behälters (1) befindet und das zum einen von zwei längs zueinander verlaufenden Seiten (2a, 2b), die jeweils einem der zwei zur Bildung des Behälters (1) zusammengesetzten Elemente (1a, 1b) zugehören, zum anderen von einer Transversalwand (3) des Behälters (1), in der eine Öffnung (10) angeordnet ist, und zum Dritten von zwei Halb-Trennwänden (7a, 8a, 9a; 7b, 8b, 9b) umgrenzt ist, die jeweils einem der zwei zur Bildung des Behälters (1) zusammengesetzten Elemente (1a, 1b) zugehören, wobei die Abdichtung des Reservoirs durch die Platte (16) erfolgt, die im zusammengebauten Zustand der zwei Elemente des Behälters (1) zwischen den beiden Halbtrennwänden (7a, 8a, 9a; 7b, 8b, 9b) eingeklemmt ist, wobei das aktive flüssige Produkt über die Öffnung (10) in das Reservoir eingeführt wird und von oben nach unten in den Rest der Platte (16) durch Kapillarwirkung diffundiert.

2. Verdampfungsvorrichtung nach Anspruch 1, dadurch gekennzeichnet, dass die Platte (16) eine Ausnehmung (17) an ihrem Rand aufweist, der derjenigen Wand (3) des Behälters zugewandt ist, welche die Öffnung (10) aufweist.

3. Vorrichtung nach einem der Ansprüche 1 und 2, dadurch gekennzeichnet, dass der Zusammenbau der zwei Elemente des Behälters (1) im wesentlichen in der Mittel-Längsebene des Behälters erfolgt.

4. Verdampfungsvorrichtung nach einem der Ansprüche 1 bis 3, dadurch gekennzeichnet, dass die Transversalwand (3) des Behälters, in der sich die Öffnung (10) befindet, von zwei Halb-Transversalwänden (3a, 3b) gebildet wird, die jeweils zu einem der beiden Elemente (1a, 1b) gehören, welche den zusammengebauten Behälter (1) bilden.

5. Verdampfungsvorrichtung nach Anspruch 4, dadurch gekennzeichnet, dass das Reservoir im Inneren des Behälters (1) die Form eines Parallelepipeds aufweist, dessen Halbtrennwände zum einen von einer Halbbande (7a oder 7b) parallel zur Halb-Transversalwand (3a oder 3b) des Behälters (1), in der die Öffnung (10) angeordnet ist und die einen bestimmten Abstand von der Halb-Transversalwand aufweist, und zum anderen von zwei Halbbanden (8a, 9a; 8b, 9b), senkrecht zu den Vorgenannten gebildet werden, welche an die Halb-Transversalwände (3a, 3b) des Behälters (1) anstossen.

6. Verdampfungsvorrichtung nach Anspruch 4, dadurch gekennzeichnet, dass in jeder Halb-Transversalwand (3a, 3b) des Behälters (1) zur Bildung der Transversalwand (3) in der sich die Öffnung (10) befindet, eine Ausnehmung (10a, 10b) vorgesehen ist, wobei diese beiden Ausnehmungen nach dem Zusammenbau der zwei Behälterelemente die Öffnung (10) zur Befüllung bilden.

7. Verdampfungsvorrichtung nach einem der Ansprüche 1 bis 6, dadurch gekennzeichnet, dass die zwei Längswände (2a, 2b) des Behälters (1) Ausnehmungen über die gesamte Oberfläche hin zur Zirkulation der Luft aufweisen, ausgenommen in dem Bereich, in dem sich das Reservoir befindet.

8. Verdampfungsvorrichtung nach Anspruch 7, dadurch gekennzeichnet, dass die Ausnehmungen der Längswände (2a, 2b) durch Jalousien bildende Lamellen gebildet sind, die schräg zur Mittelebene der Längswände verlaufen.

9. Verdampfungsvorrichtung nach einem der Ansprüche 1 bis 8, dadurch gekennzeichnet, dass die Längswände (2a, 2b) des Behälters (1) auf ihrer Innenfläche bis auf den Bereich, der das Reservoir bildet, Anschläge (11) aufweisen, auf denen die Platte (16) in Anschlag gelangt, wenn der Behälter (1) zusammengebaut wird.

10. Verdampfungsvorrichtung nach einem der Ansprüche 1 bis 9, dadurch gekennzeichnet, dass die zwei Elemente (1a, 1b) des Behälters (1) aufeinander durch Einschnappen fixiert werden, wobei die Einschnapporgane von Haken (12, 13) gebildet sind, welche miteinander zusammenwirken, wenn die Halb-Transversalwände der zwei Elemente (1a, 1b) des Behälters (1) in Kontakt miteinander kommen.

11. Verdampfungsvorrichtung nach Anspruch 10, dadurch gekennzeichnet, dass die Ränder der Halb-Transversalwände (4a, 4b; 5a, 5b; 6a, 6b) des Behälters (1) eine Vielzahl von Haken (12, 13) angeformt haben, die jeweils abwechselnd nach aussen und nach innen zum Behälter hin bzw. vom Behälter weg gewendet sind.

12. Verdampfungsvorrichtung nach einem der Ansprüche 1 bis 11, dadurch gekennzeichnet, dass die zwei Elemente (1a, 1b) des Behälters (1) entlang der Ränder der zwei Halb-Transversalwände durch ein angeformtes Filmscharnier verbunden sind, wobei die Ränder der anderen Halb-Wände, die miteinander zusammenwirken sollen, die Einschnapporgane tragen.

13. Verdampfungsvorrichtung nach einem der Ansprüche 1 bis 12, dadurch gekennzeichnet, dass das flüssige aktive Produkt eine Mischung mit insektizider Wirkung ist.

14. Vorrichtung nach einem der Ansprüche 1 bis 12, dadurch gekennzeichnet, dass das aktive Produkt eine geruchsabsorbierende Mischung ist, die eine desodorierende oder parfümierende Wirkung aufweist.

**Claims**

1. Diffuser device having, inside a receptacle (1) at least one wafer (16) of absorbent material capable of being impregnated with a liquid active product of slow evaporation, the said receptacle (1) being made of two elements (1a, 1b) which are joined to one another and which have recesses intended for ensuring the circulation of air in contact with the wafer (16) arranged inside the receptacle (1), characterized in that inside the receptacle (1) is formed a reservoir which is located in the upper part of the receptacle (1) in the filling position of the said reservoir and which is delimited, in the first place, by two opposing longitudinal walls (2a, 2b), each belonging to one of the two elements (1a, 1b) joined together to form the receptacle (1), in the second place by a transverse wall (3) of the receptacle (1), in which an orifice (10) is made, and in the third place, by two half-partitions (7a–8a–9a; 7b–8b–9b), each belonging to one of the two elements (1a, 1b) joined together to form the receptacle (1), the said reservoir being sealed off by the wafer (16) which, in the position in which the two elements of the receptacle (1) are joined together, is gripped between the two halb-partitions (7a–8a–9a; 7b–8b–9b), the liquid active product being introduced into the reservoir via the orifice (10) and diffusing from the top dwonwards into the rest of the wafer (16) as a result of capillary action.

2. Diffuser device according to Claim 1, characterized in that the wafer (16) has at its edge an indentation (17) which is adjacent to the wall (3) of the receptacle having the orifice (10).

3. Diffuser device according to either one of Claims 1 and 2, characterized in that the two elements of the receptacle (1) are joined together substantially in the longitudinal mid-plane of the said receptacle (1).

4. Diffuser device according to any one of Claims 1 to 3, characterized in that the transverse wall (3) of the receptacle, in which the orifice (10) is made, consists of two transverse half-walls (3a, 3b), each belonging to one of the two elements (1a, 1b) joined together to form the receptacle (1).

5. Diffuser device according to Claim 4, characterized in that the reservoir made inside the receptacle (1) has a parallelepipedic shape, each half-partition consisting, in the first place, of a half-strip (7a or 7b) parallel to the transverse half-wall (3a or 3b) of the receptacle (1), in which the orifice (10) is made, and located at a certain distance from this transverse half-wall and, in the second place, of two half-strips (8a, 9a; 8b, 9b) perpendicular to the preceding one and meeting the transverse half-wall (3a, 3b) of the receptacle (1).

6. Diffuser device according to Claim 4, characterized in that an indentation (10a, 10b) is provided in each transverse half-wall (3a, 3b) of the receptacle (1) intended for forming the transverse wall (3) having the orifice (10), and when the two elements of the receptacle (1) are joined together the two indentations reform the filling orifice (10).

7. Diffuser device according to any one of Claims 1 to 6, characterized in that the two longitudinal walls (2a, 2b) of the receptacle (1) have air-circulation recesses distributed over their entire surface, except in the zone where the reservoir is located.

8. Diffuser device according to Claim 7, characterized in that the recesses in the longitudinal walls (2a, 2b) consist of slats forming louvres arranged obliquely relative to the mid-plane of the said longitudinal walls.

9. Diffuser device according to any one of Claims 1 to 8, characterized in that the longitudinal walls (2a, 2b) of the receptacle (1) have on their inner face, except in the zone forming the reservoir, stops (11) on which the wafer (16) bears when the receptacle (1) is assembled.

10. Diffuser device according to any one of Claims 1 to 9, characterized in that the two elements (1a, 1b) of the receptacle (1) are joined to one another by snapping, the Snapping means consisting of hooks (12, 13) interacting with one another when the transverse half-walls of the two elements (1a, 1b) of the receptacle (1) come in contact with one another.

11. Diffuser device according to Claim 10, characterized in that the edges of the transverse half-walls (4a,4b; 5a, 5b; 6a, 6b) of the receptacle (1) are in the form of a plurality of hooks (12, 13) facing alternately towards the outside and towards the inside of the receptacle.

12. Diffuser device according to any one of Claims 1 to 11, characterized in that the two elements (1a, 1b) of the receptacle (1) are connected along the edges of two transverse half-walls by means of a moulded film hinge, and the edges of the other half-walls, which are intended to interact with one another, have snapping means.

13. Diffuser device according to any one of Claims 1 to 12, characterized in that the liquid active product is a mixture having insecticidal properties.

14. Diffuser device according to any one of Claims 1 to 12, characterized in that the active product is an odour-absorbing mixture with deodorizing or scenting properties.

FIG. 1

2 / 2

FIG. 2

FIG. 4

FIG. 3